(19) Europäisches Patentamt
European Patent Office
Office européen des brevets

(11) Publication number: **0 171 040**
**A1**

(12) # EUROPEAN PATENT APPLICATION

(21) Application number: **85109702.2**

(22) Date of filing: **02.08.85**

(51) Int. Cl.$^4$: **C 07 C 121/75**, C 09 K 19/20

(30) Priority: **06.08.84 JP 164495/84**

(43) Date of publication of application: **12.02.86**
**Bulletin 86/7**

(84) Designated Contracting States: **CH DE GB LI**

(71) Applicant: **CASIO COMPUTER COMPANY LIMITED, 6-1, 2-chome, Nishi-Shinjuku, Shinjuku-ku Tokyo (JP)**
Applicant: **SHOWA CHEMICAL CO., LTD., 1-2-2, Dojima Kita-ku, Oosaka-shi (JP)**

(72) Inventor: **Fujimura, Koh, 201 Fussa-Heights 26-1, Shimo, Fussa-shi Tokyo (JP)**
Inventor: **Kodera, Kaoru, 4-7-17, Ozone, Toyonaka-shi Oosaka (JP)**

(74) Representative: **Strasse, Joachim, Dipl.-Ing. et al, Strasse und Stoffregen European Patent Attorneys Zweibrückenstrasse 17, D-8000 München 2 (DE)**

(54) **P-Cyanobiphenylyl-p-(omega-alkoxyalkyl)l-benzoate compound and liquid crystal material.**

(57) A p-cyanobiphenylyl-[p-(ω-alkoxyalkyl)]-benzoate compound represented by a formula:

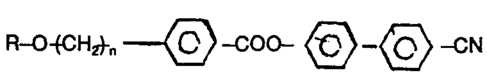

R being an alkyl group having 1–8 carbon atoms, and n being an integer of 1 to 3.

EP 0 171 040 A1

P-cyanobiphenylyl-[p-(ω-alkoxyalkyl)]-benzoate compound and liquid crystal material

The present invention relates to a p-cyanobiphenylyl-[p-(ω-alkoxyalkyl)]-benzoate compound and a liquid crystal material.

Liquid crystal display elements utilizing the electro-optic effects of liquid crystals are widely used in display devices for displaying characters, numerals and images in watches, electronic calculators and television sets. The electro-optic effect of liquid crystals include, e.g., dynamic scattering (DS) effect, twisted nematic (TN) effect, the guest-host (G-H) effect and the electrically controlled birefringence (homeotropic: DAP) effect. These electro-optic effects are utilized in various display devices in accordance with their applications. As a liquid crystal element utilizing the TN effect or G-H effect, an optical shutter is known in which transmission and shielding of light through a liquid crystal composition is controlled by an electric field.

Methods of electrically driving such a liquid crystal element include the static drive method and the dynamic drive method. An AC voltage is used as the drive voltage of liquid crystals. The two-frequency drive method is known as a voltage application method for electrically controlling a liquid crystal utilizing the dielectric scattering phenomenon of liquid crystals.

In this method, when a low-frequency voltage is applied, the liquid crystal molecular axis is aligned in the direction of an electric field. When a high-frequency voltage is applied, the liquid crystal molecular axis is aligned in a direction perpendicular to the direction of the electric field.

In a liquid crystal used in any type of liquid crystal element, predetermined characteristics such as operating temperature, response time, drive voltage or viscosity must be satisfied in accordance with an application of the element, type of electro-optic effect utilized, drive method, waveform of the application voltage and the like.

Since a single liquid crystal compound cannot satisfy such various types of characteristics, a liquid crystal material used in a liquid crystal element is usually provided by a liquid crystal mixture containing a plurality of liquid crystals each having different characteristics. Such a liquid crystal material, as a whole, satisfies required characteristics of an intended element by the complementary effects of the characteristics of the respective constituent liquid crystal compounds.

In order to widen an operating temperature range and to exhibit a liquid crystal state even at high temperatures, a liquid crystal mixture normally contains a liquid crystal compound which has a high nematic-isotropic transition point (N-I point). However, conventional liquid crystal compounds of this type which have high dielectric anisotropy ($\Delta\varepsilon$) have high viscosity or poor miscibility with other liquid crystal compounds in addition to having a relatively narrow temperature range in which they exhibit a liquid crystal state. Other conventional liquid crystal compounds which have low viscosity have small dielectric anisotropy. Thus, a liquid crystal compound having a high dielectric anisotropy, an excellent miscibility with other liquid

crystal compounds and a capacity for not rendering the overall composition highly viscous upon admixture with other liquid crystal compounds, has not previously been available.

It is, therefore, an object of the present invention to provide a compound useful as a liquid crystal having a high N-I point, a high dielectric anisotropy, an excellent miscibility with other liquid crystal compounds (low crystal-nematic transition point (C-N point)), and a capacity for not rendering the overall composition highly viscous upon admixture with other liquid crystal compounds.

It is another object of the present invention to provide a liquid crystal material which contains such a liquid crystal compound, has a wide operating temperature range and a low viscosity, and is suitable for driving by low voltages.

In order to achieve the above objects of the present invention, there is provided a p-cyanobiphenylyl-[p-(ω-alkoxyalkyl)]-benzoate compound represented by general formula (I) below:

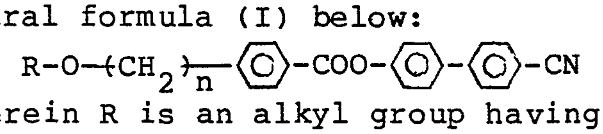 (I)

(wherein R is an alkyl group having 1 to 8 carbon atoms, and n is an integer of 1 to 3).

There is also provided, according to the present invention, a liquid crystal material comprising at least one compound represented by general formula (I).

This invention can be more fully understood from the following detailed description when taken in conjunction with the accompanying drawings, in which:

Figs. 1 to 3 show the infrared ray absorption spectra of benzoate compounds according to the present invention.

As described above, a compound of the present invention is a p-cyanobiphenylyl-[p-(ω-alkoxyalkyl)]-benzoate compound represented by general formula:

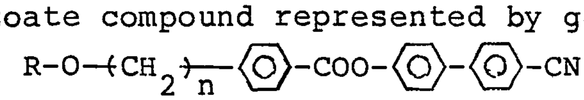 (I)

In general formula (I) above, R is an alkyl group having 1 to 8 carbon atoms and n is an integer of 1 to 3. When the number of carbon atoms of the alkyl group, represented by R, exceeds 9, the resultant compound tends to exhibit the smectic phase, tends not to exhibit the nematic phase, has a high viscosity and has low compatibility with other liquid crystals. This is inconvenient for practical application.

Preferably, R is an alkyl group having 1 to 6 carbon atoms. More preferably, R is an alkyl group (methyl group, ethyl group, propyl group or butyl group) having 1 to 4 carbon atoms. Note that n is, preferably, 2.

A compound of the present invention represented by general formula (I) can be manufactured in the following manner.

First, a ω-phenylalkyl alcohol, represented by formula:

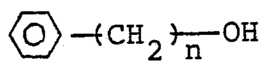

(A)

(where n has the same meaning as in general formula (I) above) is reacted with an acetylating reagent such as acetyl chloride so as to prepare 1-acetyl-4-(ω-acetyloxyalkyl)-benzene, represented by formula:

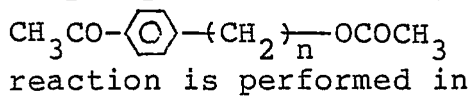

(B)

This reaction is performed in a suitable organic solvent (e.g., hexane) in the presence of a Lewis acid such as aluminum chloride, and at a low temperature of -5°C to 0°C. The acetylating reagent is used in an amount of 2 moles or more per mole of the alcohol of formula (A). The Lewis acid is used in an amount of 2 moles or more per mole of the alcohol of formula (A). Then, the compound of formula (B) is hydrolysed in the presence of sodium hydroxide to 1-acetyl-4-(ω-hydroxyalkyl)-benzene, represented by:

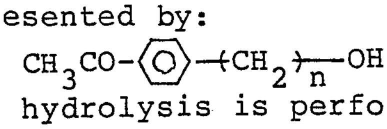

(C)

This hydrolysis is performed using a 1 to 3 normal (N) sodium hydroxide aqueous solution in an amount of

1.5 moles or more per mole of the compound of formula (B), and a temperature of 80°C to 98°C.

The compound of formula (C) is reacted with an alkylating reagent in an organic solvent such as tetrahydrofuran at a temperature of, e.g., 5 to 10°C, so as to prepare 1-acetyl-4(ω-alkoxyalkyl)-benzene, represented by:

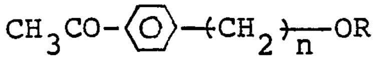 (D)

where R has the same meaning as in general formula (I). Examples of the alkylating reagent used in this reaction may include dimethyl sulfate (when R is methyl group), diethyl sulfate (when R is ethyl group), and alkyl halide (when R is an alkyl group having 3 to 8 carbon atoms). This reaction is performed in the presence of sodium hydride in an amount of 1.5 moles or more per mole of the compound of formula (c). The alkylation agent is used in an amount of 1 to 4 moles per mole of the compound of general formula (C).

Subsequently, the compound of formula (D) is oxidized using an oxidizing agent such as sodium hypochlorite and a temperature of 40 to 80°C, so as to prepare p-(ω-alkoxyalkyl)benzoic acid, represented by formula:

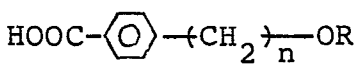 (E)

The oxidizing reagent is used in an amount of one mole or more per mole of the compound of formula (D).

Finally, the compound of formula (E) is reacted with a substantially equimolar amount of 4-cyano-4'-hydroxybiphenyl so as to obtain a compound represented by general formula (I), above. This reaction is performed in an organic solvent such as pyridine or a mixture of pyridine and ethyl acetate in the presence of dicyclohexylcarbodiimide in an amount of 1.5 moles or more per mole of the compound of formula (E) at a temperature of 25°C.

The compound of the present invention, prepared in this manner, has a high N-I point (170°C or higher),

a large dielectric anisotropy $\Delta\varepsilon$ (20 or more), and a low C-N point (130°C or lower).

Compounds of the present invention can be used as a component of liquid crystal compositions used in watches, electronic calculators, television sets, optical shutter elements, dot matrix display liquid crystal elements and 2-frequency drive liquid crystal elements. Liquid crystal compounds admixed with a compound of the present invention may include Schiff base-, azo-, azoxy-, ester-, trans-cyclohexane-, biphenyl-, dioxane- and pyrimidine-liquid crystals.

A liquid crystal material containing at least one compound according to the present invention provides a display function based on various electro-optic effects when the material is sealed between a pair of electrodes (at least one of which is transparent) and a drive voltage is applied between the electrodes. The amount of compound of general formula (I) mixed in the liquid crystal composition according to the present invention is normally 5 to 30% by weight, depending upon application and purpose of the resultant element.

The present invention will be described by way of Examples.

Example 1

<u>Preparation of Compound of General Formula (I) Wherein n = 2 and R = Ethyl Group</u>

(i) 300 mℓ of hexane were cooled to -10°C, and 195.6 g of anhydrous aluminum chloride were added thereto. 60.0 g of phenethyl alcohol were gradually added to the resultant mixture, under agitation, at the same temperature. After the addition was terminated, 116 g of acetyl chloride were added while the reaction temperature was kept at -5 to 0°C. The mixture was stirred for about 3 hours until no hydrochloric acid gas was produced. Thereafter, the reaction mixture was added to ice water and extracted with benzene. The benzene phase was washed with water. 400 mℓ of 2N

sodium hydroxide solution were added to the benzene phase, and the resultant mixture was heated to 95°C to perform hydrolysis while recovering the solvent. The reaction mixture was added to ice water to precipitate the reaction product, and the precipitate was filtered and washed with water to obtain 70 g of crude p-acetylphenethyl alcohol (n = 2 in formula (C)).

(ii) Seven grams of the p-acetylphenethyl alcohol, obtained in step (i), were dissolved in 60 mℓ of anhydrous tetrahydrofuran. 5.0 g of 50% sodium hydride were added to the solution while the temperature was kept at 5 to 10°C. Ten milliliters of diethyl sulfate were gradually added to the mixture under agitation. After the reaction temperature was raised to 50°C to terminate the reaction, the reaction mixture was extracted with benzene. After the benzene phase was washed with water, 100 mℓ of 7% aqueous solution of sodium hypochlorite was added. The temperature was raised to 80°C while recovering the benzene, thereby terminating the oxidation. Thereafter, sodium sulfite was added to consume excess sodium hypochlorite. After activated carbon was added, the mixture was filtered and the aqueous phase was neutralized with hydrochloric acid so as to provide 4.5 g of crude p-(β-ethoxyethyl)-benzoic acid (n = 2 and R = ethyl group in formula (E)).

(iii) 3.0 g of the p-(β-ethoxyethyl)-benzoic acid obtained in step (ii) above were dissolved in a solvent mixture consisting of 20 mℓ of ethyl acetate and 10 mℓ of pyridine. The temperature of the mixture was kept at 25°C, and 3.9 g of 4-cyano-4'-hydroxybiphenyl were added. While the mixture was agitated, 5.2 g of dicyclohexylcarbodiimide were added thereto and the mixture was allowed to react at the same temperature for 3 days. The mixture was then filtered and washed with benzene to separate by-produced dicyclohexylurea, and concentrate the mother liquor. Methanol was added to the concentrate to precipitate crude

p-cyanobiphenylyl-[p-(β-ethoxyethyl)]-benzoate. The crude product was dissolved in benzene. After adding silica gel, the solution was filtered and the mother liquor was concentrated. Methanol was added to the concentrate to allow crystal precipitation, and the precipitate was recrystallized from ethanol so as to obtain 2.9 g of the objective: refined p-cyanobiphenylyl-[p-(β-ethoxyethyl)]-benzoate. Fig. 1 shows the infrared ray spectrum (KBr pellet) of this final compound. The compound had a C-N point of 124°C, an N-I point of above 171°C and a dielectric anisotropy $\Delta\varepsilon$ of about 30.

Example 2

Preparation of Compound of General Formula (I) Wherein n = 2 and R = Methyl Group

A desired compound was prepared following the same procedures as in Example 1, except that an equimolar amount of dimethyl sulfate was used in place of diethyl sulfate, as used in step (ii) of Example 1. Fig. 2 shows the infrared ray spectrum (KBr pellet) of this compound. The compound had a C-N point of 125°C, an N-I point of 232°C and a dielectric anisotropy $\Delta\varepsilon$ of 20 or more.

Example 3

Preparation of Compound of General Formula (I) Wherein n = 2 and R = Butyl Group

(i) 7.0 grams of p-acetylphenethyl alcohol obtained in step (i) of Example 1 were dissolved in 60 mℓ of anhydrous tetrahydrofuran. While the solution was kept at 5 to 10°C, 4.0 g of 50% sodium hydride were added. Under agitation, 10 mℓ of n-butylbromide and 1 mℓ of hexamethyl phosphoamide were added, and the temperature was gradually raised to 20 - 25°C to terminate the reaction. Methanol was added to the reaction mixture so as to decompose excess sodium hydride. The reaction mixture was dried at a reduced pressure, and extracted with benzene. The benzene phase was washed with water

and concentrated to provide crude p-acetyl-β-n-butoxyethylbenzene. 150 mℓ of an aqueous solution of sodium hypochlorite was added to the crude product and the mixture was allowed to react at 40°C under agitation. The reaction product was washed with benzene. After sodium sulfite was added to the aqueous phase, it was treated with activated carbon. Thus, 2.5 g of crude p-(β-n-butoxyethyl)-benzoic acid were obtained.

(ii) The desired p-cyanobiphenylyl-[p-(β-n-butoxyethyl)]-benzoate was prepared following the same procedures as in step (iii) of Example 1, except that an equimolar amount of the p-(β-n-butoxyethyl)-benzoic acid in step (i) of this Example was used instead of the benzoic acid compound used in step (iii) of Example 1. Fig. 3 shows the infrared ray absorption spectrum (KBr pellet) of this compound. The compound had a C-N point of 88°C, an N-I point of above 177°C and a dielectric anisotropy Δε of about 26.

Example 4

The compounds obtained in the above Examples were mixed with other liquid crystal mixtures consisting of 30 parts by weight of 4-ethoxyphenyl-trans-4-propylcyclohexylcarboxylate, 30 parts by weight of 4-butoxyphenyl-trans-4-propylcyclohexylcarboxylate and 30 parts by weight of 4-ethoxyphenyl-trans-4-butylcyclohexylcarboxylate. The viscosity at 25°C, and the dielectric anisotropy Δε, upon application of 200 Hz voltage of each liquid crystal composition prepared in this manner, were measured. The obtained results are shown in Table 1 below.

Table 1

| Example No. of Compound Used | Viscosity (cP) | Δε |
|---|---|---|
| None | 15.3 | -1.2 |
| Example 1 | 20 | +1.9 |
| Example 3 | 22.4 | +1.5 |

As described above, the p-cyanobiphenylyl-[p-(ω-alkoxyalkyl)]-benzoate compound according to the present invention has, as a liquid crystal, a high dielectric anisotropy Δε, a wide operating temperature range in which it exhibits the liquid crystal state and a low C-N point. For this reason, the compound of the present invention has an excellent miscibility with other liquid crystals and will not, upon being mixed with other liquid crystals, increase the viscosity of the liquid crystal composition.

A liquid crystal composition using a compound according to the present invention has, owing to the above-mentioned characteristics of the compound, a wide operating temperature range and a low viscosity, despite its high dielectric anisotropy Δε, and can be operated by a low voltage (due to high dielectric anisotropy Δε).

Claims:

1. A p-cyanobiphenylyl-[p-(ω-alkoxyalkyl)]-benzoate compound represented by the general formula:

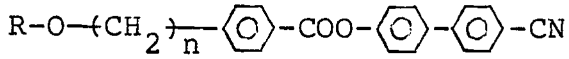

wherein R is an alkyl group having 1 to 8 carbon atoms, and n is an integer of 1 to 3.

2. A compound according to claim 1, characterized in that R is an alkyl group having 1 to 6 carbon atoms.

3. a compound according to claim 2, characterized in that n is 2.

4. A compound according to claim 1, characterized in that R is an alkyl group having 1 to 4 carbon atoms, and n is 2.

5. A compound according to claim 4, represented by the formula:

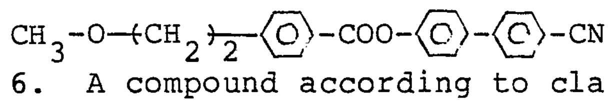

6. A compound according to claim 4, represented by the formula:

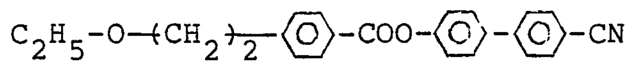

7. A compound according to claim 4, represented by the formula:

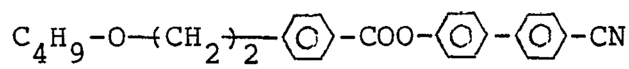

8. A liquid crystal material comprising at least one p-cyanobiphenylyl-[p-(ω-alkoxyalkyl)]-benzoate compound, represented by the general formula:

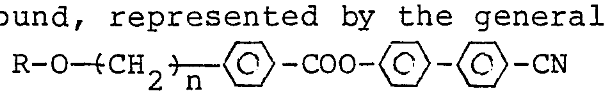

wherein R is an alkyl group having 1 to 8 carbon atoms, and n is an integer of 1 to 3.

9. A liquid crystal material according to claim 8, characterized in that R is an alkyl group having 1 to 6 carbon atoms.

10. A liquid crystal material according to claim 9, characterized in that n is 2.

11. A liquid crystal material according to claim 8, characterized in that R is an alkyl group having 1 to 4 carbon atoms, and n is 2.

12. A liquid crystal material according to claim 11, characterized in that the benzoate compound is represented by the formula:

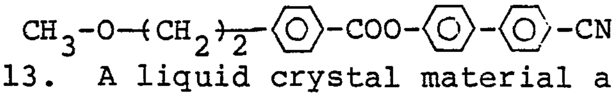

13. A liquid crystal material according to claim 11, characterized in that the benzoate compound is represented by the formula:

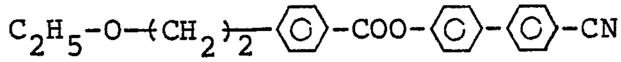

14. A liquid crystal material according to claim 11, characterized in that the benzoate compound is represented by:

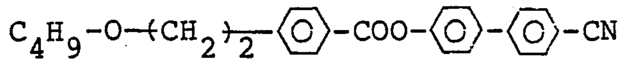

15. A liquid crystal material according to claim 8, characterized in that the benzoate compound has an N-I point of not lower than 170°C.

16. A liquid crystal material according to claim 8, characterized in that the benzoate compound has a C-N point of not higher than 130°C.

17. A liquid crystal material according to claim 8, characterized in that the benzoate compound has a dielectric anisotropy not lower than 20.

18. A liquid crystal material according to claim 8, containing at least one liquid crystal selected from the group consisting of Schiff base-, azo-, azoxy-, ester-, trans-cyclohexane-, biphenyl-, dioxane- and pyrimidine-liquid crystals.

FIG. 1

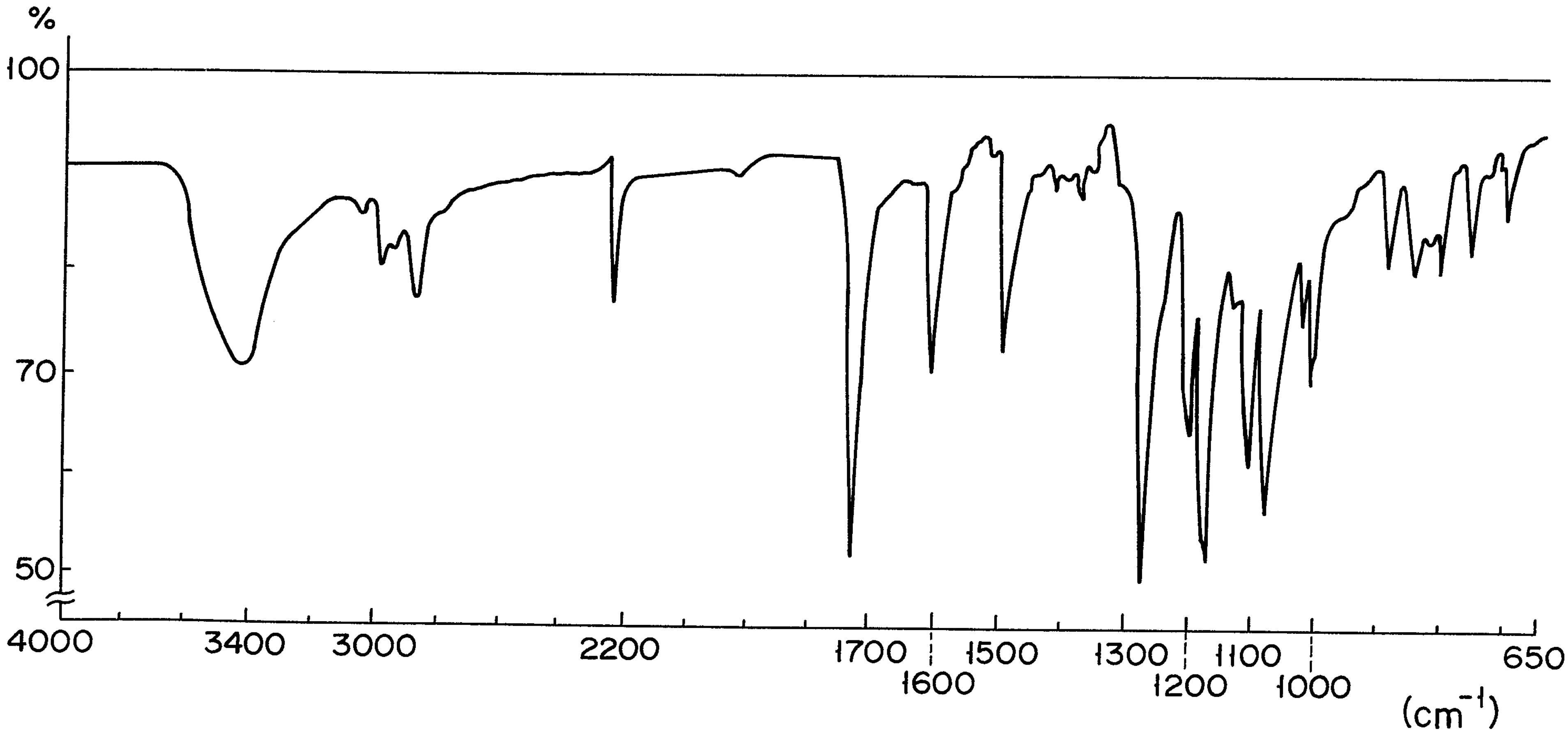
%
100
70
50
4000
3400
3000
2200
1700
1600
1500
1300
1200
1100
1000
650
(cm⁻¹)

FIG. 2

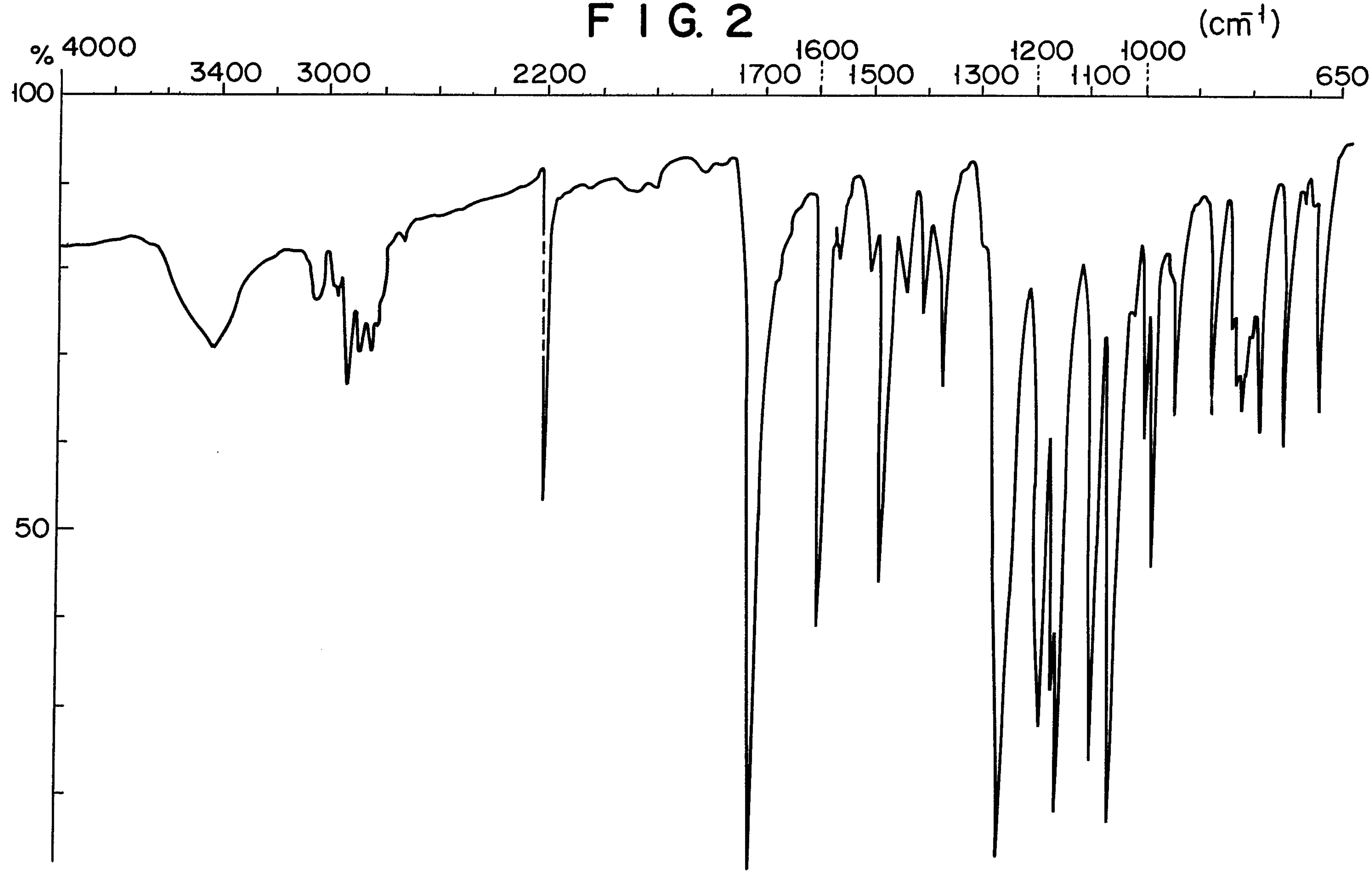
(cm⁻¹)
%
100
50
4000
3400
3000
2200
1700
1600
1500
1300
1200
1100
1000
650

F I G. 3

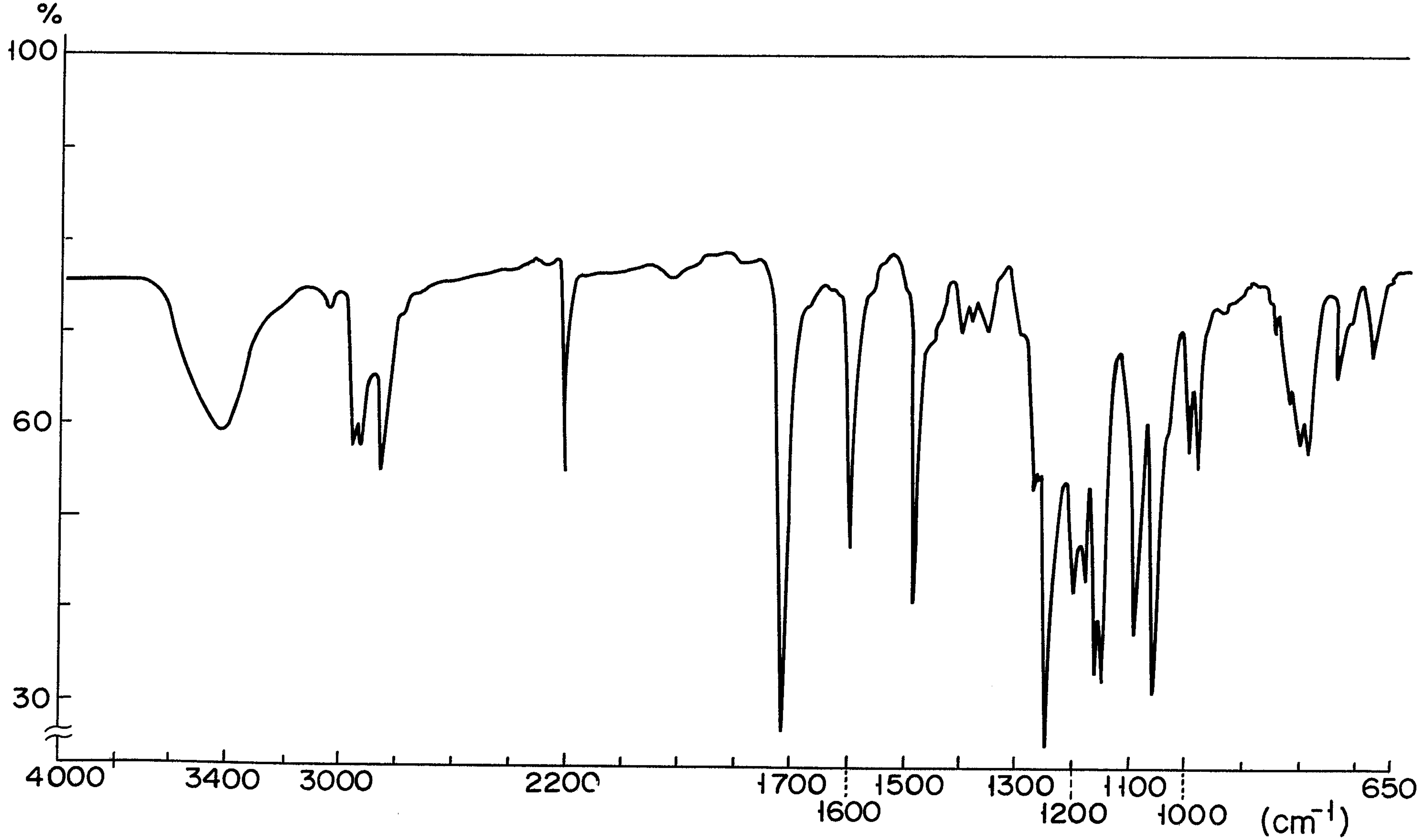

%
100
60
30
4000
3400
3000
2200
1700
1600
1500
1300
1200
1100
1000
650
(cm-1)

European Patent Office

0 171 040

# EUROPEAN SEARCH REPORT

EP 85 10 9702

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | | | C 07 C 121/75<br>C 09 K 19/20 |
| Y | DE-A-2 613 293 (MERCK)<br>* Claims 1,4,9,13; page 10 *<br>--- | 1-18 | |
| X,Y | EP-A-0 058 981 (HITACHI AND KANTO CHEMICAL)<br>* Claims 1-7 *<br>--- | 1-18 | |
| A | PATENTS ABSTRACTS OF JAPAN, vol. 8, no. 145 (C-232)[1582], 6th July 1984; & JP - A - 59 53 459 (ASAHI GLASS K.K.) 28.03.1984<br>* Abstract *<br>--- | 1-18 | |
| A | PATENTS ABSTRACTS OF JAPAN, vol. 6, no. 197 (C-128)[1075], 6th October 1982; JP - A - 57 108 056 (CHISSO K.K.) 05.07.1982<br>* Abstract *<br>--- | 1-18 | TECHNICAL FIELDS SEARCHED (Int. Cl.4) |
| A | PATENTS ABSTRACTS OF JAPAN, vol. 6, no. 158 (C-120)[1036], 19th August 1982; & JP - A - 57 77 658 (CHISSO K.K.) 15.05.1982<br>* Abstract *<br>----- | 1-18 | C 07 C 121/00<br>C 09 K 19/00 |

The present search report has been drawn up for all claims

| Place of search | Date of completion of the search | Examiner |
|---|---|---|
| THE HAGUE | 17-09-1985 | THEUNS H.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82